# EUROPEAN PATENT APPLICATION

(11) **EP 1 466 974 A1**
(43) Date of publication of application: **13.10.2004**
(21) Application number: 03008470.1
(22) Date of filing: 11.04.2003
(51) Int. Cl.: C12N 15/10, A01K 67/00, A01K 67/027

(54) **Inducible site-directed mutagenesis through conditional gene rescue**

(71) Applicant: MAX-DELBRÜCK-CENTRUM FÜR MOLEKULARE MEDIZIN, 13125 Berlin (DE)
(72) Inventor: Gotthardt, Michael, 10435 Berlin (DE); Radke, Michael, 13187 Berlin (DE)
(74) Representative: Krauss, Jan B., Dr.

(57) **Abstract**

The present invention relates to a conditionally inducible site-directed mutant cell, comprising a mutated allele of a gene; wherein said allele comprises a mutation that was introduced by using a suitable mutagenesis technique, a rescue allele of said mutated gene that can be conditionally inactivated, wherein said mutation in said mutated allele of said gene interferes with survival and/or causes an adverse phenotype, such as temporal and/or local phenotypes, such as cell cycle-specific, cell-type specific, tissue-specific, protein-expression specific, tissue-development specific, organ-specific, organ-development-specific and/or embryonic lethal phenotypes. According to further aspects thereof, the present invention relates to a conditionally inducible site-directed mutant cell culture, tissue, organ, or non-human embryo, comprising a cell and a respective non-human organism, in particular a genetically deficient or Knock-out-mammal, -rodent, -nematode, -fish, -plant or -insect. Finally the invention provides a method for inducible site-directed mutagenesis through conditional gene rescue, either in vitro or in vivo.

## Description

The present invention relates to a conditionally inducible site-directed mutant cell, comprising a mutated allele of a gene; wherein said allele comprises a mutation that was introduced by using a suitable mutagenesis technique, a rescue allele of said mutated gene that can be conditionally inactivated, wherein said mutation in said mutated allele of said gene interferes with survival and/or causes an adverse phenotype, such as temporal and/or local phenotypes, such as cell cycle-specific, cell-type specific, tissue-specific, protein-expression specific, tissue-development specific, organ-specific, organ-development-specific and/or embryonic lethal phenotypes. According to further aspects thereof, the present invention relates to a conditionally inducible site-directed mutant cell culture, tissue, organ, or non-human embryo, comprising a cell and a respective non-human organism, in particular a genetically deficient or Knock-out-mammal, -rodent, -nematode, -fish, -plant or -insect. Finally the invention provides a method for inducible site-directed mutagenesis through conditional gene rescue, either *in vitro,* in tissue culture, or *in vivo.*

### Background of the invention

Mutants are essential tools for research in molecular biology. There is a therefore a constant need for new mutagenesis techniques in the art that allow for the production of new mutants, and, in particular, mutants that contain mutations in genes, alleles and/or regions of the chromosome that could not be mutated before.

Due to the fact that some genes are essential for the vital functions of a cell or tissue, not all genes or regions of a genome can be inactivated or modified without causing adverse effects that might interfere with analysis. Nevertheless, some of the inactivations or functional modifications of genes or regions are lethal only during early stages of the development of an organism. These genes cause, for example, a class of development-specific phenotypes, the so-called "embryo-lethal" phenotypes. Other genes cause lethal phenotypes transiently during the later phases of development, i.e. temporal and/or local phenotypes, such as cell cycle-specific, cell-type specific, tissue-specific, tissue-development specific, organ-specific, organ-development-specific and/or embryonic lethal phenotypes.

A vast number of mutagenesis techniques are known in the art. These include mutagenesis techniques employing vector systems, irradiation, random integration of foreign DNA, site specific recombination, homologous recombination, and/or chemical mutagenesis. Using these techniques, mutations such as deletions, point mutations, insertions, inversions, and the like, can be introduced. In addition, the introduction of modem high-throughput-technology into mutagenesis allows for the rapid generation of an enormous number of mutants in relatively short time.

With respect to mutagenesis in the mouse, the following articles can be taken as examples; Russ A, et al. Random mutagenesis in the mouse as a tool in drug discovery. Drug Discov Today 2002 Dec 1;7(23):1175-83; Schimenti J, Bucan M. Functional genomics in the mouse: phenotype-based mutagenesis screens. Genome Res 1998 Jul;8(7):698-710; and van der Weyden L, Adams DJ, Bradley A. Tools for targeted manipulation of the mouse genome. Physiol Genomics 2002 Dec 3;11(3):133-64.

One of the more recently established mutagenesis techniques is the so-called "conditional knock-out technology" that is used in eukaryotic organisms. Examples are, in addition to chemically and/or nutritionally conditional mutants (for example in Arabidopsis thaliana, yeast but also bacteria), mutants based on site-specific recombinase-activities, such as the site-specific recombinase FLP (e.g. in yeast; Michel S, et al. Generation of conditional lethal Candida albicans mutants by inducible deletion of essential genes. Mol Microbiol 2002 Oct;46(1):269-80; and in mice, e.g. Rodriguez CI, et al. High-efficiency deleter mice show that FLPe is an alternative to Cre-loxP. Nat Genet 2000 Jun;25(2):139-40) and the frequently used Cre/loxP recombination system (e.g. in mice; Pluck A. Conditional mutagenesis in mice: the Cre/loxP recombination system. Int J Exp Pathol 1996 Dec;77(6):269-78; Kwan KM. Conditional alleles in mice: practical considerations for tissue-specific knockouts. Genesis 2002 Feb;32(2):49-62; Robertson A, et al. Effects of mouse strain, position of integration and tetracycline analogue on the tetracycline conditional system in transgenic mice. Gene 2002 Jan 9;282(1-2):65-74, and in plant cells: United States Patent 5,658,772).

Furthermore the so-called "first-generation mouse tumour models", which used transgenic mice or conventional knockouts, are now being superseded by models that are based on conditional knockouts and mice that carry regulatable oncogenes. In these mice, somatic mutations can be induced in a tissue-specific and time-controlled fashion, which more faithfully mimics, for example, sporadic tumour formation. These second-generation models provide exciting new opportunities to gain insight into the contribution of known and unknown genes in the initiation, progression and treatment of, for example, cancer, and mimic human cancer better than ever before (Jonkers J, Berns A. Conditional mouse models of sporadic cancer. Nat Rev Cancer 2002 Apr;2(4):251-65).

So far, the conditional knockout technology has only been used in order to bypass embryonic lethality as in the tissue specific knockout of the titin m-line segment (Gotthardt M, Hammer RE, Hubner N, Monti J, Witt CC, McNabb M, Richardson JA, Granzier H, Labeit S, Herz J. Conditional expression of mutant M-line titins results in cardiomyopathy with altered sarcomere structure. J Biol Chem 2003 Feb 21;278(8):6059-65.) or to reconstitute functionality of a tissue such as the placenta (Wu L, De Bruin A, Saavedra HI, Starovic M, Trimboli A, Yang Y, Opavska J, Wilson P, Thompson JC, Ostrowski MC, Rosol TJ, Woollett LA, Weinstein M, Cross JC, Robinson ML, Leone G. Extra-embryonic function of Rb is essential for embryonic development and viability. Nature. 2003 Feb 27;421(6926):942-7.).

Currently, the smallest modification that can be introduced into a genomic region of choice using conditional knock-out technology is the deletion of a single exon. Mutations of single bases that cause a lethal phenotype can not be temporally and/or locally regulated, in order to provide an adult organism that would be present for analysis. In addition, for the production of multiple inducible knock-outs of a single gene, one conditionally inducible targeting vector must be generated for each modification. The systems as present therefore lack the flexibility that would be required for more specific generation of mutants as well as their mutational analyses.

It is therefore an object of the present invention, to provide for novel conditional mutants. It is another object of the present invention, to provide for an easy, fast and convenient conditional mutagenesis technique that allows for the introduction of mutations at the exon and even sub-exon level in genes in which a mutated allele of said gene interferes with survival and/or causes an adverse phenotype.

According to a first aspect of the present invention, this object is solved by a conditionally inducible site-directed mutant cell, comprising a) a mutated allele of a gene; wherein said allele comprises a mutation that was introduced by using a suitable mutagenesis technique, and b) a rescue allele of said mutated gene that can be conditionally inactivated, wherein said mutation in said mutated allele of said gene interferes with survival and/or causes an adverse phenotype.

The term "wherein said mutation in said mutated allele of said gene interferes with survival and/or causes an adverse phenotype" shall mean that the mutation (or mutations) lead to a phenotype that, at some point during the development of the mutated cell (either being isolated or as part of a tissue, organ and/or organism), will either lead to the death of the cell, inhibit the growth of the cell or lead to a developmental disorder of the cell. Furthermore, "adverse phenotype(s)" are phenotypes such as slower growth of the cell, auxotrophy to certain nutritional factors, repair defects, transformation of the cell, cell surface modifications, temperature dependent phenotypes, error-prone transcription and/or translation, telomer-shortening, chromatin-related disorders, apoptosis, cell cycle disorders, and the like. The phenotypes can either be encoded directly by said gene (i.e. the mutated gene product and/or its mutated regulatory regions) or be due to a disturbed interaction of the mutated gene product with other components of the cell. Another possibility would be disturbed production of signal molecules that are excreted (e.g. hormones). Finally, the mutation can be tissue-specific.

The "rescue allele" according to the present invention allows for the development and/or survival of the organism and contains, in one embodiment, the second allele that bypasses the mutation of the mutated allele. In the "simplest" case, the rescue allele is an unmutated copy of the mutated allele, nevertheless, the rescue allele could also be mutated in a manner that suppresses the effect of the mutated allele. The rescue allele according to the present invention can be regulatably inactivated, both locally (e.g. tissue-specific) and/or temporally (e.g. development-specific), leading to the expression of the mutated allele of said gene. According to the present invention, only the rescue allele must be introduced by a conditional targeting vector, whilst all other mutations can be introduced using conventional mutagenesis techniques. This results in an increased and superior flexibility of the system according to the present invention, in particular in the case of multiple exon-level as well as sub-exon-level mutations to be introduced in the allele to be mutated. One example would be mutations in both the gene and the respective regulatory regions, either leading to no expression, increased expression ("overexpression") or substrate specific expression ("inducible" expression).

The reconstitution used by Wu et al. (2003, see above) nicely demonstrates that the rescue approach is suitable to circumvent or ameliorate an undesired phenotype. Other than in the present invention, Wu et al. only use a "rescue" in order to derive a functional tissue (placenta), a feature that was accomplished independently by tetraploid aggregation and chimeric embryos. In contrast to their approach, the present invention follows a strategy to bypass adverse phenotypes to allow for expression of a mutated allele in an adult animal.

In a preferred embodiment of the conditionally inducible site-directed mutant cell according to the present invention, said mutated allele of said gene comprises a mutation at the exon or sub-exon level, such as a deletion, point mutation, insertion, inversion, and the like. These mutations can be introduced into the allele to be mutated using any conventional mutagenesis technique and depend from the desired size and position of the mutation(s) to be introduced. Suitable mutagenesis technique comprise, for example, a vector system, transposon mutagenesis, irradiation, random integration of foreign DNA, site specific recombination, homologous recombination, and/or chemical mutagenesis. One example is random mutagenesis, i.e. an introduction of one or more mutations at random positions of the parent enzyme (i.e., as opposed to site-specific mutagenesis). Suitable techniques for introducing random mutations are by use of a suitable physical or chemical mutagenising agent, by use of a suitable oligonucleotide, or by subjecting the DNA sequence to PCR generated mutagenesis. Furthermore, the random mutagenesis may be performed by use of any combination of these mutagenising agents. The mutagenising agent may, e.g., be one which induces transitions, transversions, inversions, scrambling, deletions, and/or insertions. Examples of a physical or chemical mutagenising agent suitable for the present purpose includes ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues.

When such agents are used the mutagenesis is typically performed by incubating the DNA sequence encoding the allele to be mutagenised in the presence of the mutagenising agent of choice under suitable conditions for the mutagenesis to take place, and selecting for mutated DNA having the desired properties.

When the mutagenesis is performed by the use of an oligonucleotide, the oligonucleotide may be doped or spiked with the three non-parent nucleotides during the synthesis of the oligonucleotide at the positions wanted to be changed. The doping or spiking may be done so that codons for unwanted amino acids are avoided. The doped or spiked oligonucleotide can be incorporated into the DNA encoding the allele by any published technique using e.g., PCR, LCR or any DNA polymerase and ligase. When PCR generated mutagenesis is used either a chemically treated or non-treated gene encoding a parent allele is subjected to PCR under conditions that increases the misincorporation of nucleotides (Deshler 1992, Leung et al. 1989).

A mutator strain of E. coli (Fowler et al. 1974), S. cerevisiae or any other microbial organism may be used for the random mutagenesis of the DNA encoding the allele by e.g., transforming a plasmid containing the parent enzyme into the mutator strain, growing the mutator strain with the plasmid and isolating the mutated plasmid from the mutator strain. The mutated plasmid may subsequently be transformed into the expression organism. The DNA sequence to be mutagenised may conveniently be present in a genomic or cDNA library prepared from an organism expressing the unmutated allele enzyme. Alternatively, the DNA sequence may be present on a suitable vector such as a plasmid or a bacteriophage, which as such may be incubated with or otherwise exposed to the mutagenizing agent. The DNA to be mutagenised may also be present in a host cell either by being integrated in the genome of said cell or by being present on a vector harboured in the cell. Finally, the DNA to be mutagenised may be in isolated form. It will be understood that the DNA sequence to be subjected to random mutagenesis is preferably a cDNA or a genomic DNA sequence.

In yet another preferred embodiment of the conditionally inducible site-directed mutant cell according to the present invention, the rescue allele and/or its transcription product(s) comprise(s) recombination target sites, such as, for example, lox or FRT sites, sites for the attachment of antisense oligonucleotides, for example DNA, PNA and/or RNA-oligonucleotides, sites for ribozyme activities, and or sites that interfere with specific siRNA for expression. All the above features of the rescue allele are present in order to allow for an inducible inactivation of the rescue allele, i.e. a "silencing" of the expression, blocking of transcription, blocking of translation or blocking the activity of the gene product. In another particular embodiment of the conditionally inducible site-directed mutant cell according to the present invention, said rescue allele comprises a conditionally inducible genetic construct which either directly or via its expression product inhibits the function of any non-mutated copy of said mutated allele.

In another embodiment, the conditionally inducible site-directed mutant cell according to the present invention can contain multiple mutated alleles of genes and/or a multiply mutated allele of a gene together with their suitable rescue allele(s). Again, this combination can only be achieved due to the increased flexibility of the mutagenesis system of the present invention. Commonly used knock-out techniques are either conditional or generate "regular" mutations below the exon-level. A convenient combination of these different techniques is not possible, thus, multiple conditional mutagenesis would require multiple conditional targeting vectors. This requirement is efficiently bypassed by the present invention. The feature of combining the two techniques is now possible with the present invention, furthermore facilitating the creation of multiple conditional targeting vectors.

According to another aspect of the present invention, a conditionally inducible site-directed mutant cell is provided, wherein said allele to be mutated encodes for titin (Gotthardt M, Hammer RE, Hubner N, Monti J, Witt CC, McNabb M, Richardson JA, Granzier H, Labeit S, Herz J. Conditional expression of mutant M-line titins results in cardiomyopathy with altered sarcomere structure. J Biol Chem 2003 Feb 21;278(8):6059-65.). In general, suitable genes are known to the person of skill in the art and can be easily identified in the respective scientific literature and databases. Examples of the wide variety of genes that cause, for example, embryonic lethal phenotypes are Rnf2 (Voncken JW, et al. Rnf2 (Ringlb) deficiency causes gastrulation arrest and cell cycle inhibition. Proc Natl Acad Sci USA 2003 Mar 4;100(5):2468-73); Cbfbeta (Kundu M, et al. Cbfbeta interacts with Runx2 and has a critical role in bone development. Nat Genet 2002 Dec;32(4):639-44); and VEGF (Carmeliet P, et al. Insights in vessel development and vascular disorders using targeted inactivation and transfer of vascular endothelial growth factor, the tissue factor receptor, and the plasminogen system. Ann N Y Acad Sci 1997 Apr 15;811:191-206). Of course, these genes only represent a very small choice of possible genes.

In another aspect of the present invention, a conditionally inducible site-directed mutant cell is provided, wherein said interference with survival and/or adverse phenotype is selected from temporal and/or local phenotypes, such as cell cycle-specific, cell-type specific, tissue-specific, protein-expression specific, tissue-development specific, organ-specific, organ-development-specific and/or embryonic lethal phenotypes. Examples of suitable genes and phenotypes can be found in the respective literature. In case of tissue-specific alleles in the mouse, the following articles, in connection with others, might be referred to: Kwan KM. Conditional alleles in mice: practical considerations for tissue-specific knockouts. Genesis 2002 Feb;32(2):49-62; Robertson A, et al. Effects of mouse strain, position of integration and tetracycline analogue on the tetracycline conditional system in transgenic mice. Gene 2002 Jan 9;282(1-2):65-74; Hsieh JC, et al. Mesd encodes an LRP5/6 chaperone essential for specification of mouse embryonic polarity. Cell 2003 Feb 7;112(3):355-67; Chang H, Lau AL, Matzuk MM. Studying TGF-beta superfamily signaling by knockouts and knockins. Mol Cell Endocrinol 2001 Jun 30;180(1-2):39-46; Robertson EJ, et al. Use of embryonic stem cells to study mutations affecting postimplantation development in the mouse. Ciba Found Symp 1992;165:237-50; discussion 250-5; Lendahl U. Transgenic analysis of central nervous system development and regeneration. Acta Anaesthesiol Scand Suppl 1997;110:116-8; Aasrum M, Prydz H. Gene targeting of tissue factor, factor X, and factor VII in mice: their involvement in embryonic development. Biochemistry (Mosc) 2002 Jan;67(1):25-32; Stec DE, Sigmund CD.; Modifiable gene expression in mice: kidney-specific deletion of a target gene via the cre-loxP system. Exp Nephrol 1998 Nov-Dec;6(6):568-75; and Chapman RS, et al. The role of Stat3 in apoptosis and mammary gland involution. Conditional deletion of Stat3. Adv Exp Med Biol 2000;480:129-38.

Preferably, the conditionally inducible site-directed mutant cell according to the present invention can be selected from a prokaryotic cell, a eukaryotic cell, a diploid cell, a plant cell, a mammalian cell, a nematode cell, a fish cell, an insect cell, and, in particular, a non-human stem-cell. One preferred example would be a mouse stem-cell (see, for example, Robertson EJ, et al. Use of embryonic stem cells to study mutations affecting postimplantation development in the mouse. Ciba Found Symp 1992;165:237-50; discussion 250-5).

According to yet another aspect of the present invention, a conditionally inducible site-directed mutant cell culture, tissue, organ, or non-human embryo, comprising a cell according to the present invention is provided. Even more preferably, the invention provides for a conditionally inducible site-directed mutant non-human organism, in particular a genetically deficient or Knock-out-mammal (such as a goat or sheep), -rodent (such as a rabbit, mouse, rat or hamster), -nematode (such as Caenorhabditis elegans), -fish (such as zebrafish), -plant (such as Arabidopsis thaliana, corn, rice or potato), -insect or jellyfish, comprising a cell, a culture, tissue or organ according to the present invention. According to yet another embodiment of the present invention the conditionally inducible site-directed mutant non-human organism according to the invention contains multiple mutated alleles of genes and/or a multiply mutated allele of a gene together with their suitable rescue allele(s). The present invention therefore provides for convenient multiple conditional mutations-containing animals.

According to yet another embodiment of the present invention, a conditionally inducible site-directed mutant non-human organism is provided, wherein the interference with survival and/or adverse phenotype is selected from temporal and/or local phenotypes, such as cell cycle-specific, cell-type specific, tissue-specific, tissue-development specific, protein-expression specific, organ-specific, organ-development-specific and/or embryonic lethal phenotypes. Examples for these phenotypes can be easily obtained from the literature in the field and/or are as defined above.

According to another important aspect thereof, the present invention provides for a method for producing an inducible site-directed mutant cell capable of conditional gene rescue, wherein said method comprises a) introducing in a target cell a mutated allele of a gene to be mutated by using a suitable mutagenesis technique, b) introducing in said target cell a rescue allele of said gene that can be conditionally inactivated, and c) optionally, cultivating said target cell under conditions that allow for a selection of cells that contain both the mutated allele and the rescue allele of said gene, wherein said mutation in said mutated allele of said gene interferes with survival and/or causes an adverse phenotype. Thus, the method of the present invention is used in order to introduce a mutation (or mutations) that lead to a phenotype that, at some point during the development of the mutated cell (either being isolated or as part of a tissue, organ and/or organism), will either lead to the death of the cell, inhibit the growth of the cell or lead to a developmental disorder of the cell.

Preferred is a method according to the present invention, wherein the cell to be mutated is selected from a prokaryotic cell, a eukaryotic cell, a diploid cell, a plant cell, a mammalian cell, a fish cell, a nematode cell, an insect cell, and, in particular, a non-human stem-cell.

Furthermore, adverse phenotype(s), i.e. phenotypes such as slower growth of the cell, auxotrophy to certain nutritional factors, repair defects, transformation of the cell, cell surface modifications, temperature dependent phenotypes, error-prone transcription and/or translation, telomer-shortening, chromatin-related disorders, apoptosis, cell cycle disorders, and the like can be introduced. The phenotypes can either be encoded directly by said gene (i.e. the mutated gene product and/or its mutated regulatory regions) or be due to a disturbed interaction of the mutated gene product with other components of the cell. Another possibility would be disturbed production of secreted products of a cell, for example, signal molecules that are excreted (e.g. hormones). Finally, the mutation can be introduced tissue-specifically.

Preferred is a method according to the present invention, wherein the suitable mutagenesis technique comprises introducing a mutation at the exon or sub-exon level, such as a deletion, point mutation, insertion, inversion, and the like, preferably by using a suitable mutagenesis technique employing a vector system, irradiation, transposon mutagenesis, random integration of foreign DNA, site specific recombination, homologous recombination, and/or chemical mutagenesis. All these mutagenesis techniques are well-known to the person skilled in the art and can be found in the standard literature. Some of the methods are also described above. The mutation(s) can be either introduced in situ in vivo, for example, in the DNA of a living cell or in vitro, using, e.g. DNA recombinant techniques.

Both the mutated allele and/or the rescue allele can be introduced into the desired host (a cell, tissue, organ and/or organism) using standard techniques in the art. These include transformation (chemical and/or physical), transfection, lipofection, particle gun, transduction, electroporation, and the like. Some examples of such methods are described in US 6,503,755 "Particle transfection: rapid and efficient transfer of polynucleotide molecules into cells", US 6,320,030 "Mucin-biomolecules complex for transfection", US 5,928,944 "Method of adenoviral-mediated cell transfection", US 5,633,156 "Methods for calcium phosphate transfection", US 5,627,159 "Enhancement of lipid cationic transfections in the presence of serum", US 5,593,875 "Methods for calcium phosphate transfection, and US 5,024,939 "Transient expression system for producing recombinant protein".

In one particularly preferred embodiment of the method according to the present invention, introducing said rescue allele comprises transfection or infection of the cell with a rescue allele genetic construct comprising recombination target sites, e.g. lox or FRT sites, sites for the attachment of antisense oligonucleotides, e.g. DNA, PNA and/or RNA-oligonucleotides, sites for ribozyme activities (see, for example, Sioud M. Nucleic acid enzymes as a novel generation of anti-gene agents. Curr Mol Med 2001 Nov;1(5):575-88), and/or sites that interfere with specific siRNA for expression (see, for example, Shi Y. Mammalian RNAi for the masses. Trends Genet 2003 Jan;19(1):9-12). All the above features of the rescue allele are present in order to allow for an inducible inactivation of the rescue allele, i.e. a "silencing" of the expression, blocking of transcription, blocking of translation or blocking the activity of the gene product. In another particular embodiment of the method according to the present invention, introducing said rescue allele comprises transfer of a conditionally inducible genetic construct into the cell, which either directly or via its expression product inhibits the function of any non-mutated copy of said mutated allele. One example for such an inhibiting expression product could be a promoter specific repressor that specifically recognises the promoter of the non-mutated copy of said mutated allele.

Particularly preferred is a method according to the present invention, wherein a tissue-specific rescue allele and/or mutated allele is introduced into the cell, tissue, organ and/or organism. Examples of such genes include genes that are specific for tissues in skin, colon, heart, muscle, brain, lung, epithelium in general, liver, prostate, breast, spleen, lymph nodes, and nasopharynx, but also leaves, roots, pollen, and flowers.

After the desired allele(s) have been introduced in the respective organism, using either in vivo or in vitro methods, the resulting mutants are selected for the desired phenotypes. The required selection methods depend from the type of mutation to be introduced and/or any selection marker that might be employed. Common selection methods comprise chemical selection (e.g. antibiotic resistance markers), temperature sensitivity, and the like. Nevertheless, the mutated allele itself might be used for the selection.

The desired mutant cells, tissues, and/or organisms are then cultured, in order to either propagate the mutants and/or for further production of the final mutant organisms. Common culture methods include regular medium broth culture, fermenter culture, tissue culture, greenhouse culture, animal farming and marine culture. Based on the raised material and or organisms, heterozygotes containing both the mutated allele(s) and the rescue allele(s) can be produced by suitable crossing and selection of, for example, non-human animals or plants.

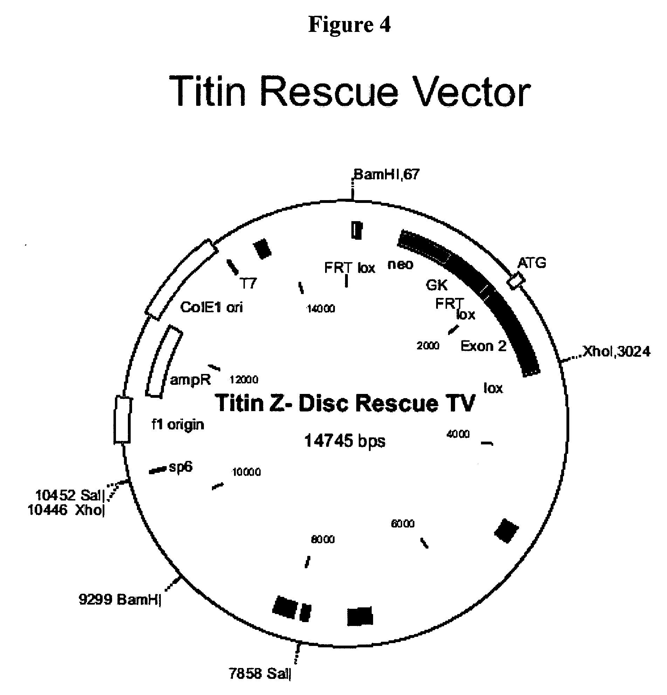

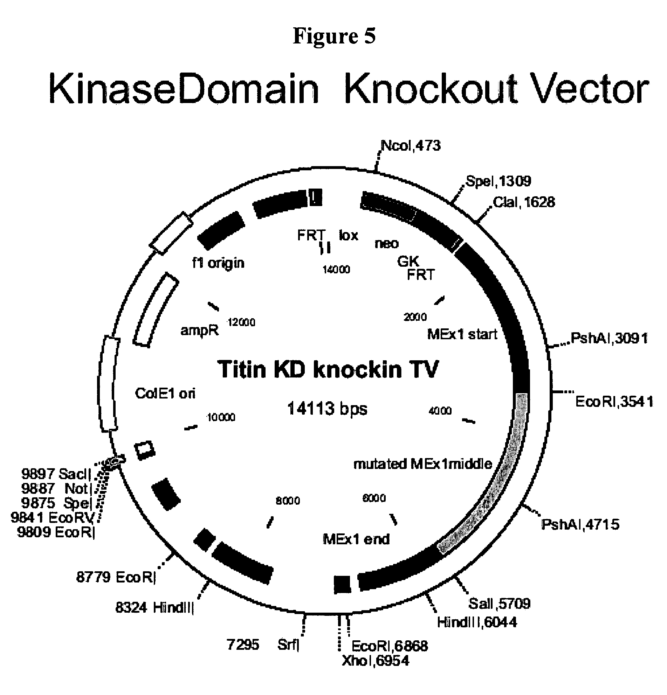

According to another aspect of the method of the present invention, a conditionally inducible site-directed mutant cell is provided, wherein said allele to be mutated encodes for titin (Gotthardt M, Hammer RE, Hubner N, Monti J, Witt CC, McNabb M, Richardson JA, Granzier H, Labeit S, Herz J. Conditional expression of mutant M-line titins results in cardiomyopathy with altered sarcomere structure. J Biol Chem 2003 Feb 21;278(8):6059-65.). In general, suitable genes are known to the person of skill in the art and can be easily identified in the respective scientific literature and databases. Other examples are depicted in Figures 4 and 5 herein.

According to yet another embodiment of the method according of the present invention, the method comprises the introduction of multiple mutated alleles of genes and/or a multiply mutated allele of a gene together with their suitable rescue allele(s) in order to generate a conditionally inducible site-directed mutant non-human organism according to the invention that contains multiple mutated alleles of genes and/or a multiply mutated allele of a gene together with their suitable rescue allele(s).

According to another aspect of the method according to the present invention, said interference with survival and/or adverse phenotype is selected from temporal and/or local phenotypes, such as cell cycle-specific, cell-type specific, tissue-specific, tissue-development specific, organ-specific, organ-development-specific and/or embryonic lethal phenotypes. Examples for these phenotypes can be easily obtained from the literature in the field and/or are as defined above.

According to another important aspect thereof, the present invention provides for a method for producing an inducible site-directed mutant cell capable of conditional gene rescue, wherein said method further comprises the step of d) conditionally inactivating said rescue allele of said gene to be mutated by using a suitable inactivation technique. Said inactivation of course depends on the particulate conditional rescue allele(s) that is/are used.

In a preferred method according to the present invention, conditionally inactivating said rescue allele of said gene to be mutated by using a suitable inactivation technique comprises a technique selected from site directed recombination, such as cre/lox or Flp/FRT inactivation, antisense inactivation using oligonucleotides, e.g. DNA, PNA and/or RNA-oligonucleotides, RNA-interference, such as ribozyme activity inactivation, siRNA expression-inactivation, inactivation of the gene product (protein) and/or its activity and/or inducible inactivation of the non-mutated allele, such as through antibodies, inactivation of the activity of a fusion protein or induced proteolysis. Similarly to the earlier steps of the method according to the present invention, the step of inactivation can also be performed in vivo and/or in vitro.

According to one further preferred embodiment of the present invention, said cell is present in a tissue, organ, non-human embryo or non-human organism, in particular a mammal, rodent, nematode, fish, plant, or insect, as described above.

According to yet another aspect of the present invention, a method for the production of an inducible site-directed non-human mutant-organism capable of conditional gene rescue is provided, comprising a) generating an inducible site-directed mutant cell employing the method according to the present invention as described above, and b) generating a non-human mutant organism comprising said mutant cell(s). Said generation can be performed using conventional techniques in the art, which comprise regular medium broth culture, fermenter culture, tissue culture, greenhouse culture, animal farming and marine culture. Based on the raised material and or organisms, heterozygotes containing both the mutated allele(s) and the rescue allele(s) can be produced by suitable crossing and selection of, for example, non-human animals or plants. Examples of non-human mutant organisms that contain said mutant cell or tissue or organ of the present invention are particular a mammal, such as a cow, horse, camel, goat, sheep, pig, cat, dog, a rodent, such as a mouse, rat, hamster, guinea-pig a nematode, such as Caenorhabditis elegans, a fish, such as zebrafish, salmon, herring, a plant, such as corn, cotton, tobacco, rice, potato, rape, coconut, wheat, rye, hop, plum, apple, arabidopsis, a moss, or an insect, such as drosophila melanogaster.

All references as cited herein are incorporated in their entirety. The present invention shall now be further described based on the accompanying Figures and Examples, without being limited thereto.

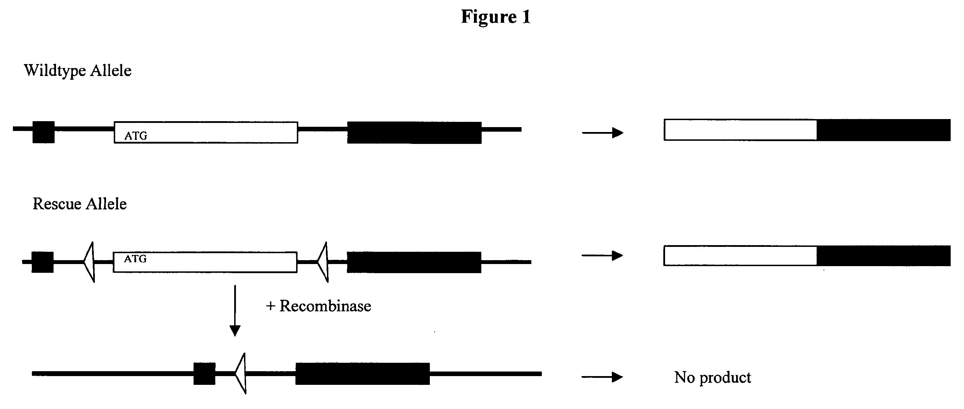

- Figure 1:: Schematic representation of an embodiment of the mechanisms involved in the inducible position-specific mutagenesis by conditional gene-rescue. The rescue allele allows for the conditional inhibition of the expression of an allele (recombined allele - no product)

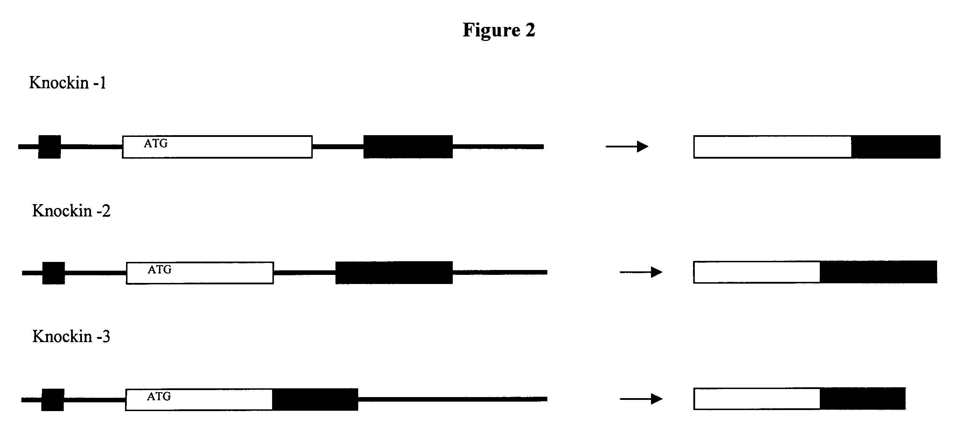

- Figure 2:: Another schematic representation of an embodiment of the mechanisms involved in the inducible position-specific mutagenesis by conditional gene-rescue. Different deletions can be produced by conventional targeting vectors.

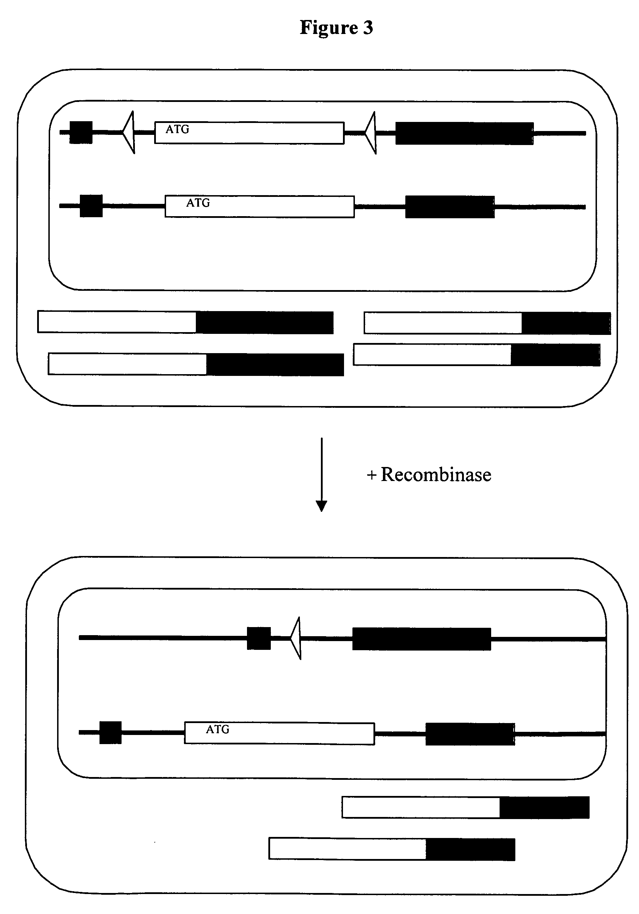

- Figure 3:: Schematic representation of the embodiment of the mechanisms involved in the inducible position-specific mutagenesis by conditional gene-rescue *in vivo*, wherein the mutated allele is titin. Double heterozygotic cells having a Knockin -1 allele and a rescue allele after recombination produce only mutated titin that is derived from the mutated allele.
- Figures 4 and 5:: Schematic representations of two vectors that are used in the examples for the generation of titin mutants according to the present invention.

### Examples

As an example for a tissue-specific gene, the gene titin has been mutated and a conditionally inactivated rescue allele has been constructed. Both constructs are then used to generate a transgenic mouse model.

Titin is a giant protein responsible for muscle elasticity and provides a scaffold for several sarcomeric proteins, including the novel titin-binding protein MURF-1, which binds near the titin M-line region. Another unique feature of titin is the presence of a serine/threonine kinase-like domain at the edge of the M-line region of the sarcomere, for which no physiological catalytic function has yet been shown. Previously, the exons MEx1 and MEx2 (encoding the kinase domain plus flanking sequences) had been conditionally deleted at different stages of embryonic development (Gotthardt et al., see above), showing an important role for MEx1 and MEx2 in early cardiac development (embryonic lethality) as well as postnatally when disruption of M-line titin leads to muscle weakness and death at approximately 5 weeks of age. Myopathic changes include pale M-lines devoid of MURF-1, and gradual sarcomeric disassembly.

### Example 1: Cloning of the knock-in vector to introduce the targeted deletion.

A targeting construct was assembled by standard procedures using long range genomic PCR (LA PCR 2.1 from Takara). A 1.5 kb fragment containing M-line Exon 2 was subcloned into a plasmid containing a FRT-site flanked neomycine resistance gene. The long arm (7 kb) contains 4 kb 5' of M-line Exon 1 and a deletion mutant of M-line Exon 1 that was engineered by PCR-based gene assembly to lack titin's MURF-1 binding site.

The targeting vector was verified by sequence analysis of all exons, the M-line Exon 1 deletion, and the proper integration of the neomycin resistance cassette into the intron 3' of M-line Exon 1.

### Example 2: Cloning of the knock-in vector to introduce the targeted mutations.

Additional knock-in vectors with mutations of titin's kinase active site were constructed using the knock-in vector lacking titin's MURF-1 binding site by exchanging M-line Exon 1 without the MURF-1 binding site to a M-line Exon-1 using unique restriction sites within M-line Exon 1. The kinase-site included within the M-line Exon 1 internal fragment was mutagenized using the quick-change kit from Stratagene according to the manufacturer's instructions.

### Example 3: Construction of the rescue vector.

A targeting vector to introduce a rescue allele was assembled from a mouse genomic BAC clone (bacterial artificial chromosome library MGS1 from mouse ES cells; Genome Systems/Incyte Genomics) spanning the 5' region of the mouse titin gene. A PCR-based strategy was used to introduce neomycin expression cassette flanked by IoxP- and FRT-sites into the Intron 5' of Exon 2, which contains the ATG. A Iox-site was inserted 3' of Exon 2. The targeting vector was verified by sequencing.

### Example 4: Generation of targeted ES cells and animal models carrying the mutation deletion, and rescue construct.

Homologous recombination in Embryonic Stem Cells was performed after electroporation of the linearized targeting vector and selection with G418. Individual colonies were analyzed by PCR and southern blot. Positive clones were used to derive chimeric animals as described in Willnow, TE and Herz, J (1994) *Methods Cell Biol* **43 Pt A**, 305-334 and Gotthardt, M., Hammer, RE, Hubner, N, Monti, J, Witt, CC, McNabb, M, Richardson, JA, Granzier, H, Labeit, S and Herz, J (2003) *J Biol Chem* **278**, 6059-6065. For knock-in- and rescue-vectors the intronic neo cassette has the potential to affect the phenotype of knockout animals. Therefore, heterozygous animals were mated that contained the altered titin locus to transgenic mice that expressed the Flp recombinase in their germline (Dymecki, SM (1996) *Proc Natl Acad Sci* USA **93**, 6191-6196). Offspring from this mating in which the neo cassette had been removed by Flp-mediated excision was used to generate a colony of homozygous mice that only contained the IoxP and FRT sequences (rescue vector) or the mutation and a residual FRT site (knock-in vector).

Animals containing the rescue allele were mated to the MCKcre or MHCcre transgenic animals in order to establish founder lines that were subsequently bread to the various knock-in mutants to derive double heterozygous animals with the Cre transgene, that were used for analysis.

Genotyping (PCR and Southern Blotting). Genomic DNA from embryonic and postnatal mouse tails or yolk sacs was genotyped essentially as described previously (Gotthardt, M., Hammer, RE, Hubner, N, Monti, J, Witt, CC, McNabb, M, Richardson, JA, Granzier, H, Labeit, S and Herz, J (2003) *J Biol Chem* **278**, 6059-6065). Primers were designed to detect homologous recombination, presence of the 5' IoxP site and Flp, and Cre-mediated recombination. For Southern genotyping, genomic DNA was digested with the appropriate restriction endonucleases and probed with a fragment outside of the short arm according to standard procedures (Willnow, TE and Herz, J (1994) *Methods Cell Biol* **43 Pt A,** 305-334).

### Example 5: Deletion of the rescue allele in tissue culture using Si-RNA.

To investigate the function of titin in cardiomycocytes we established primary cultures as published by Rust et al. (Rust, EM, Albayya, FP and Metzger, IM (1999) *J Clin Invest* **103**, 1459-1467). Mice containing the heterozygous knock-in alleles were used to derive the cells. Si-RNA was used to specifically interfere with expression of the wildtype allele, while leaving expression of the mutated allele unchanged. Design and application of Si-RNA was performed as described by Elbashir et al. (Elbashir, SM, Harborth, J, Ledeckel, W, Yalcin, A, Weber, K, and Tuschl, T (2001) *Nature* **411**, 494-498).

### Example 6: RNAi in the mouse germline.

shRNA expression vectors were constructed and transferred to mouse ES-cells by electroporation as described by Carmell et al. (Carmell, MA, Zhang, L, et al. (2003) Nature Structural Biology 10(2), 91-92). Mice that express shRNA in sufficient amounts to downregulate the rescue allele were mated with titin knockin-animals to obtain double heterozygous animals.

## Claims

1. A conditionally inducible site-directed mutant cell, comprising
a) a mutated allele of a gene; wherein said allele comprises a mutation that was introduced by using a suitable mutagenesis technique,
b) a rescue allele of said mutated gene that can be conditionally inactivated,
wherein said mutation in said mutated allele of said gene interferes with survival and/or causes an adverse phenotype.

2. The conditionally inducible site-directed mutant cell according to claim 1, wherein said mutated allele of said gene comprises a mutation at the exon or sub-exon level, such as a deletion, point mutation, insertion, inversion, and the like.

3. The conditionally inducible site-directed mutant cell according to claim 1 or 2, wherein said rescue allele and/or its transcription product(s) comprises recombination target sites, e.g. lox or FRT sites, sites for the attachment of antisense oligonucleotides, e.g. DNA, PNA and/or RNA-oligonucleotides, sites for ribozyme activities, and or sites that interfere with specific siRNA for expression.

4. The conditionally inducible site-directed mutant cell according to claim 1 or 2, wherein said rescue allele comprises a conditionally inducible genetic construct which either directly or via its expression product inhibits the function of any non-mutated copy of said mutated allele.

5. The conditionally inducible site-directed mutant cell according to any of claims 1 to 4, containing multiple mutated alleles of genes and/or a multiply mutated allele of a gene together with their suitable rescue allele(s).

6. The conditionally inducible site-directed mutant cell according to any of claims 1 to 5,
wherein said allele encodes for titin.

7. The conditionally inducible site-directed mutant cell according to any of claims 1 to 6,
wherein said interference with survival and/or adverse phenotype is selected from temporal and/or local phenotypes, such as cell cycle-specific, cell-type specific, tissue-specific, protein-expression specific, tissue-development specific, organ-specific, organ-development-specific and/or embryonic lethal phenotypes.

8. The conditionally inducible site-directed mutant cell according to any of claims 1 to 7, which is selected from a prokaryotic cell, a eukaryotic cell, a diploid cell, a plant cell, a mammalian cell, a nematode cell, a fish cell, an insect cell, and, in particular, a non-human stem-cell.

9. A conditionally inducible site-directed mutant cell culture, tissue, organ, or non-human embryo, comprising a cell according to any of claims 1 to 8.

10. A conditionally inducible site-directed mutant non-human organism, in particular a genetically deficient or Knock-out-mammal, -rodent, -nematode, -fish, -plant or -insect, comprising a cell according to any of claims 1 to 8 or a culture, tissue or organ according to claim 9.

11. The conditionally inducible site-directed mutant non-human organism according to claim 10, containing multiple mutated alleles of genes and/or a multiply mutated allele of a gene together with their suitable rescue allele(s).

12. The conditionally inducible site-directed mutant non-human organism according to claim 9 or 10, wherein said interference with survival and/or adverse phenotype is selected from temporal and/or local phenotypes, such as cell cycle-specific, cell-type specific, tissue-specific, tissue-development specific, protein-expression specific, organ-specific, organ-development-specific and/or embryonic lethal phenotypes.

13. A method for producing an inducible site-directed mutant cell capable of conditional gene rescue, comprising
a) introducing in a target cell a mutated allele of a gene to be mutated by using a suitable mutagenesis technique,
b) introducing in said target cell a rescue allele of said gene that can be conditionally inactivated, and
c) optionally, cultivating said target cell under conditions that allow for a selection of cells that contain both the mutated allele and the rescue allele of said gene,
wherein said mutation in said mutated allele of said gene interferes with survival and/or causes an adverse phenotype.

14. The method according to claim 13, wherein said suitable mutagenesis technique comprises introducing a mutation at the exon or sub-exon level, such as a deletion, point mutation, insertion, inversion, and the like, preferably by using a suitable mutagenesis technique employing a vector system, irradiation, random integration of foreign DNA, site specific recombination, homologous recombination, and/or chemical mutagenesis.

15. The method according to claim 13 or 14, wherein introducing said rescue allele comprises transfection or infection of the cell with a rescue allele genetic construct comprising recombination target sites, e.g. lox or FRT sites, sites for the attachment of antisense oligonucleotides, e.g. DNA, PNA and/or RNA-oligonucleotides, sites for ribozyme activities, and or sites that interfere with specific siRNA for expression.

16. The method according to claim 13 or 14, wherein introducing said rescue allele comprises transfer of a conditionally inducible genetic construct into the cell, which either directly or via its expression product inhibits the function of any non-mutated copy of said mutated allele.

17. The method according to any of claims 13 to 16, wherein a tissue specific rescue allele and/or mutated allele is introduced.

18. The method according to any of claims 13 to 17, wherein said allele encodes for titin.

19. The method according to any of claims 13 to 18, wherein said cell is selected from a prokaryotic cell, a eukaryotic cell, a diploid cell, a plant cell, a mammalian cell, a fish cell, a nematode cell, an insect cell, and, in particular, a non-human stem-cell.

20. The method according to any of claims 13 to 19, comprising the introduction of multiple mutated alleles of genes and/or a multiply mutated allele of a gene together with their suitable rescue allele(s).

21. The method according to any of claims 13 to 20, wherein said interference with survival and/or adverse phenotype is selected from temporal and/or local phenotypes, such as cell cycle-specific, cell-type specific, tissue-specific, tissue-development specific, organ-specific, organ-development-specific and/or embryonic lethal phenotypes.

22. The method according to any of claims 13 to 20, further comprising
d) conditionally inactivating said rescue allele of said gene to be mutated by using a suitable inactivation technique.

23. The method according to claim 22, wherein conditionally inactivating said rescue allele of said gene to be mutated by using a suitable inactivation technique comprises a technique selected from site directed recombination, such as cre/lox or Flp/FRT inactivation, antisense inactivation using oligonucleotides, e.g. DNA, PNA and/or RNA-oligonucleotides, RNA-interference, such as ribozyme activity inactivation, siRNA expression-inactivation, inactivation of the gene product (protein) and/or its activity and/or inducible inactivation of the non-mutated allele, such as through antibodies, inactivation of the activity of a fusion protein or induced proteolysis.

24. The method according to any of claims 13 to 23, wherein said method is performed in vivo or in vitro.

25. The method according to any of claims 13 to 24, wherein said cell is present in a tissue, organ, non-human embryo or non-human organism, in particular a mammal, rodent, nematode, fish, plant, or insect.

26. A method for the production of an inducible site-directed non-human mutant-organism capable of conditional gene rescue, comprising
a) generating an inducible site-directed mutant cell according to the method according to any of claims 13 to 24, and
b) generating a non-human mutant organism comprising said mutant cell.

27. An inducible site-directed non-human mutant-organism, produced according to claim 26, in particular a mammal, nematode, rodent, fish, plant, or insect.
